# EUROPEAN PATENT APPLICATION

(11) **EP 3 301 171 A1**
(43) Date of publication of application: **04.04.2018**
(21) Application number: 17190955.9
(22) Date of filing: 13.09.2017
(51) Int. Cl.: C12M 1/12, C12M 1/00

(54) **METHOD FOR CUTTING CELL SHEET**

(30) Priority: 28.09.2016 JP 2016189727
(71) Applicant: Shibuya Corporation, Kanazawa-shi, Ishikawa 920-8681 (JP)
(72) Inventor: TAKAHASHI, Masayo, Wako-shi, Saitama 351-0198 (JP); KOIDE, Naoshi, Wako-shi, Saitama 351-0198 (JP); ONISHI, Takuya, Kanazawa-shi, Ishikawa 920-8681 (JP); SASAKI, Motoi, Kanazawa-shi, Ishikawa 920-8681 (JP); EJIRI, Masanari, Kanazawa-shi, Ishikawa 920-8681 (JP)
(74) Representative: Hodsdon, Stephen James

(57) **Abstract**

The present invention relates to a method for cutting a cell sheet S, which includes cutting the cell sheet S that is cultured in the inside of a container 4 which has accommodated a culture medium M therein, by a cutting device 3 that emits a laser L.

The cutting method includes:
removing the culture medium M from the container 4 and also sealing the container 4 by a lid portion 13 provided with a transmission part made from a material which allows the laser L to pass through, in the inside of an aseptic room 2 that has been kept in an aseptic condition;
taking out the container 4 sealed by the lid portion 13, from the aseptic room 2, and moving the container to the cutting device 3 that is provided in the outside of the aseptic room 2; and
in the cutting device 3, passing the laser L through the transmission part of the lid portion 13, and cutting the cell sheet S by the laser L.

The method enables the cell sheet S to be cut in such a state that the aseptic condition is kept.

## Description

### BACKGROUND OF THE INVENTION

### Field of the Invention

The present invention relates to a method for cutting a cell sheet, and specifically relates to a cutting method for cutting a cell sheet by a laser.

### Description of the Related Art

In order to use a cultured cell sheet for examination or treatment, a cell sheet has been conventionally cut into a required size, and a method using a laser is known as such a method for cutting the cell sheet (International Publication No. WO 2012/115244, particularly, in column No. 0050).

As for a cutting device that is used in the method for cutting the cell sheet using the laser, a cutting device is known that has a holding table which holds a culture container that accommodates the above described cell sheet therein, laser irradiation means which emits the above described laser, and moving means which relatively moves the above described holding table and the laser (National Publication of International Patent Application No. 2005-534941).

Here, when the cell sheet is cut by the laser as in the above described International Publication No. WO 2012/115244, it is necessary to move a culture container into the above described cutting device, but there has been such a problem that foreign substances, microorganisms and the like result in attaching to the cell sheet in the above described culture container at this time.

With respect to such problems, the present invention provides a method for cutting the cell sheet, which can cut the cell sheet in a state of having kept an aseptic condition.

### SUMMARY OF THE INVENTION

Specifically, a method for cutting a cell sheet according to the invention in claim 1 is a method of moving a container that accommodates the cell sheet therein to a cutting device that emits a laser, and cutting the cell sheet in the container by the laser, characterized in that the method includes:
sealing the container by a lid portion provided with a transmission part made from a material which allows the laser to pass through, in the inside of an aseptic room that has been kept in an aseptic condition;
taking out the container sealed by the lid portion from the aseptic room, and moving the container into the cutting device that is provided in the outside of the aseptic room; and
in the cutting device, passing the laser through the transmission part of the lid portion, and cutting the cell sheet by the laser.

The method according to the invention in the above described claim 1 includes sealing the culture container by the lid portion in the inside of an isolator, and thereby can cut the cell sheet in a state of having kept the inside of the culture container in the aseptic condition.

At this time, a transmission part is provided in the lid portion, which is made from a material that allows the laser to pass through. Thereby, the laser passes through the transmission part, and it becomes possible to cut the cell sheet by the laser.

### BRIEF DESCRIPTION OF THE DRAWINGS

FIG. 1 is a view showing a cell sheet cutting system according to the present example;
FIG. 2 is a view showing a culture container according to the present example;
FIG. 3 is a view showing a method for cutting the cell sheet;
FIG. 4 is a view showing the method for cutting the cell sheet; and
FIG. 5 is a view showing a culture container having another form.

### DETAILED DESCRIPTION OF THE PREFERRED EMBODIMENTS

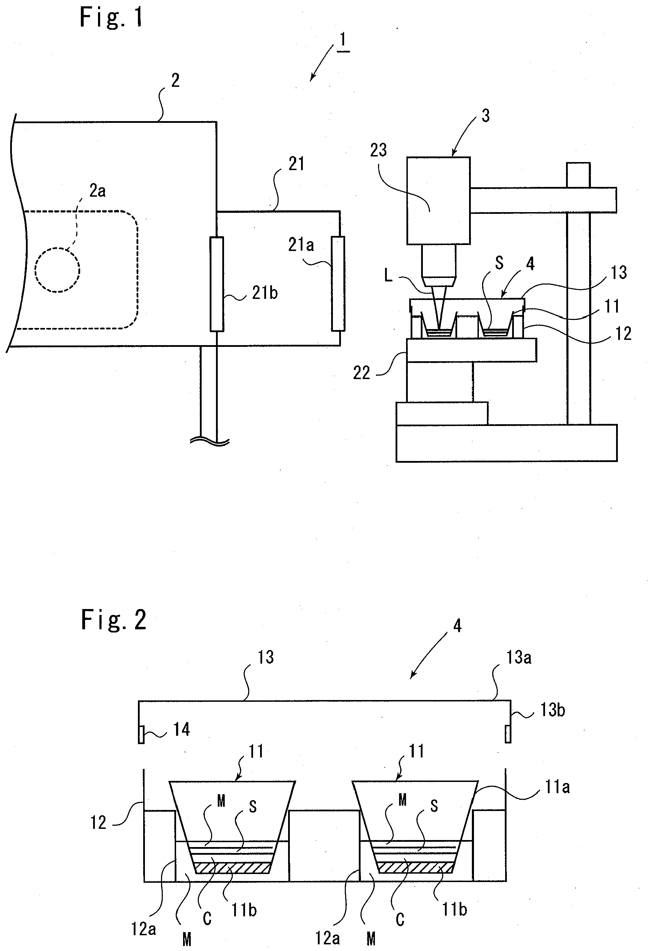

An illustrated example will be described below. FIG. 1 shows a cell sheet cutting system 1, which cuts a cultured cell sheet S into a required size. The system 1 includes: an isolator 2 that works as an aseptic room of which the inside is kept in an aseptic condition; and a cutting device 3 which cuts the above described cell sheet S by a laser L.

The cell sheet S to be discussed in the present example can include, for instance, a cell sheet formed of a retinal pigment epithelial cell described in International Publication No. WO 2012/115244, and a cell sheet S formed of other tissues.

As for the above described isolator 2 and cutting device 3, conventionally known ones can be used. The inside of the isolator 2 is kept in the aseptic condition by unillustrated air cleaning means, and in contrast to this, the above described cutting device 3 is provided in an external space outside the isolator 2.

The above described isolator 2 and cutting device 3 are installed in an environment as in a clean room or the like, but the environment is not an environment as in the internal space of the isolator 2, in which the aseptic condition is kept, and foreign matters, microorganisms and the like may float therein.

For this reason, the cell sheet cutting system 1 is configured so as to conduct a required operation for the cell sheet S under the aseptic environment of the above described isolator 2, and cut the cell sheet S in the above described cutting device 3 in a state of having kept the aseptic condition by using the following culture container 4; and can prevent foreign matters and microorganisms from attaching to the cell sheet S when cutting the cell sheet S.

FIG. 2 shows the culture container 4 that accommodates the above described cell sheet S therein. The culture container 4 includes inner containers 11 that accommodate the cell sheet S therein, and an outer container 12 that accommodates a plurality of inner containers 11 and also accommodates culture media M. In addition, the culture container 4 of the present example is configured so that the internal space is sealed by a lid portion 13.

The above described inner container 11 includes a cylindrical portion 11a that is formed in a tapered shape and is made from a resin, and a membrane 11b that is provided at the bottom face of the cylindrical portion 11a and is made from a resin; and is configured so that the above described cell sheet S is cultured in the upper part of the above described membrane 11b.

The above described membrane 11b has innumerable through holes which are not illustrated, and is configured so that the above described culture medium M can flow through the through holes thereof.

In addition, the above described membrane 11b is configured so that a collagen gel layer C which is formed of collagen is formed on the upper face thereof, when the above described cell sheet S is cultured, and so that the cell sheet S is cultured on the upper face of the collagen layer.

The above described outer container 12 is formed from a material which allows the laser L to pass through and also has a bottomed box shape; has a plurality of recessed supporting portions 12a for supporting the above described inner containers 11 formed in the bottom, respectively; and is configured so as to be capable of culturing a plurality of cell sheets S by the plurality of inner containers 11.

The above described supporting portions 12a are each configured so as to accommodate the culture medium M therein, and the inner container 11 which is supported by the supporting portion 12a is configured so that the above described membrane 11b is separated upward from the bottom face of the above described outer container 12.

Due to such a structure, the culture medium M that is accommodated in each of the supporting portions 12a can flow to the inside of the inner container 11 from the above described gap through the through holes formed in the above described membrane 11b.

The above described lid portion 13 is mounted on an opening that is formed in the upper part of the above described outer container 12, and includes: an upper face portion 13a which covers the opening of the above described outer container 12; a side portion 13b which is provided on the outer periphery of the upper face portion 13a; and sealing means 14 which is provided between the side portion 13b and the outer container 12.

The above described sealing means 14 is configured so as to come in close contact with the outer peripheral surface of the outer container 12, when the above described lid portion 13 closes the above described outer container 12, and prevent foreign matters, microorganisms and the like from contaminating the inside of the culture container 4.

In addition, the upper face portion 13a in the above described lid portion 13 constitutes a transmission part which is formed from a material that allows the laser L to pass through, such as a resin such as polystyrene, acrylic, glass and quartz glass. Incidentally, only a part of the above described upper face portion 13a may be formed to be the transmission part, or the whole of the lid portion 13 may be formed from a material which constitutes the transmission part.

The above described isolator 2 has a glove 2a which an operator wears for performing an operation in the inside of the isolator 2, and a pass box 21 which is used for carrying a material and the like into and out form the isolator 2, provided on its side face.

The above described pass box 21 is provided with a carry-in/out door 21a that can free the inside to the outside, and a connection door 21b that is provided between the above described isolator 2 and itself; and the pass box 21 is also provided with decontamination means using hydrogen peroxide vapor and the like, though the decontamination means is not illustrated.

When the material and the like are carried in the above described isolator 2, the material is carried into the pass box 21 from the above described carry-in/out door 21a in such a state that the above described connection door 21b is closed, and the material and the like in the pass box 21 are decontaminated in such a state that the carry-in/out door 21a is closed.

After that, the above described connection door 21b is opened and the material is moved into the isolator 2. Thereby, it becomes possible to move the material into the isolator 2 in such a state that the foreign matters, microorganisms and the like have been decontaminated which have attached to the material and the like in the external space.

In addition, the isolator 2 is provided with an incubator for culturing the cell sheet S, so as to be contactable/separable with/from the isolator, though the incubator is not illustrated. The incubator is configured so as to accommodate the above described cell sheet S together with the above described culture container 4 therein, and culture the cell at a position separated from the isolator 2.

The above described isolator 2 and the incubator are configured so as to be contactable/separable with/from each other in such a state that the aseptic condition has been kept, and so as to be capable of moving the culture container 4 between the isolator 2 and the incubator in an open state in which the above described lid portion 13 is removed.

The above described cutting device 3 includes: a holding table 22 for holding the above described culture container 4 thereon; laser irradiation means 23 for emitting the laser L; and unillustrated moving means that relatively moves the above described holding table 22 and the laser irradiation means 23. Incidentally, such the cutting device 3 itself is conventionally known as is disclosed in National Publication of International Patent Application No. 2005-534941.

The above described holding table 22 is configured so as to be capable of mounting the culture container 4 thereon on which the above described lid portion 13 is mounted, and the above described moving means is configured so as to move the culture container 4 in a horizontal direction together with the holding table 22.

The above described laser irradiation means 23 is configured so as to emit the laser L downward, and is configured so that the laser L emitted by the means transmits the transmission part which constitutes the lid portion 13 mounted on the above described culture container 4, and irradiates the above described cell sheet S which is accommodated inside.

On the other hand, due to the above described moving means which moves the culture container 4, the above described cell sheet S could cut into a required shape by the laser L. At this time, the collagen gel layer C which holds the cell sheet S is also cut by the laser L.

The laser L which has cut the above described cell sheet S and the collagen gel layer C is controlled to transmit through the above described membrane 11b and the bottom face of the outer container 12, and to be adsorbed by the above described holding table 22.

Here, it is desirable that the wavelength of the laser L which is emitted by the above described laser irradiation means 23 is in a range of 300 nm to 500 nm. When the wavelength is set at less than 300 nm, the laser L cannot pass through the above described lid portion 13, and when the wavelength is set so as to exceed 500 nm, the laser becomes unsuitable for micromachining.

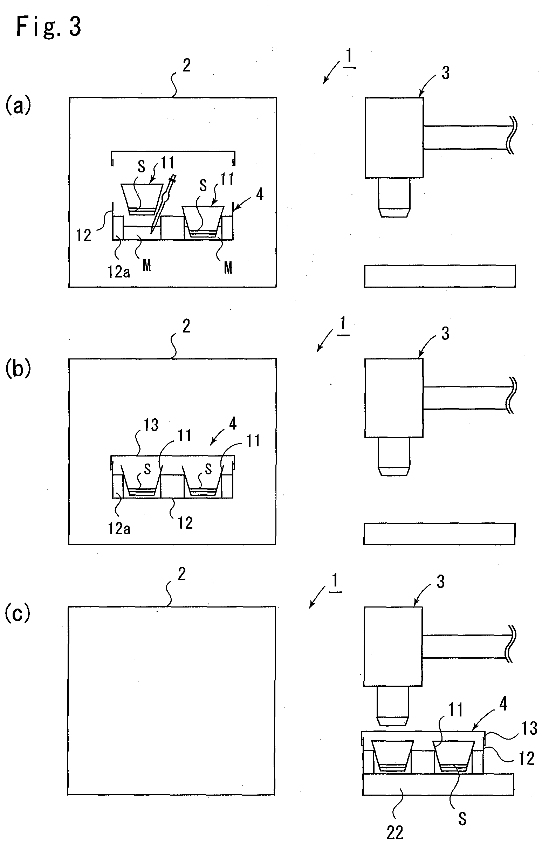

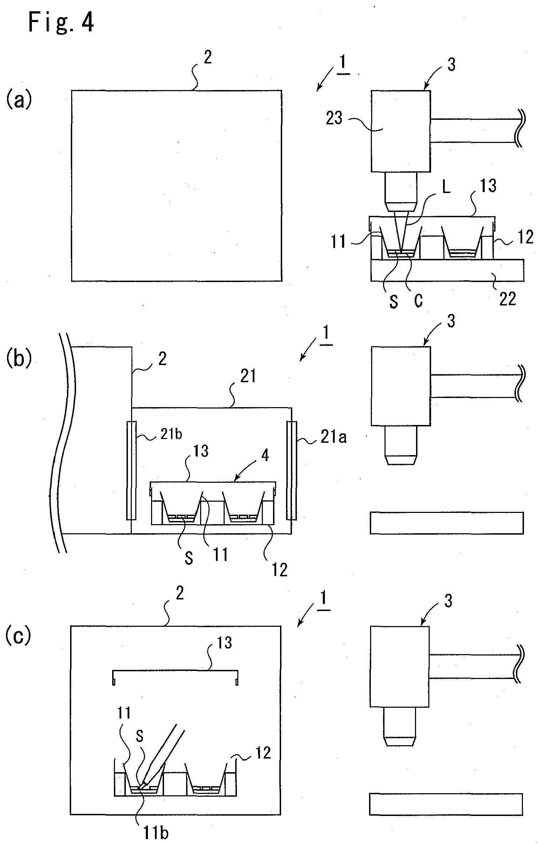

A method for cutting the above described cell sheet S by the cell sheet cutting system 1 having the above described structure will be described below with reference to FIG. 3 and FIG. 4.

The cell sheet S is cultured in the culture container 4 in advance. Specifically, in the above described inner container 11 of the culture container 4, cells are seeded on the upper face of the collagen gel layer C formed on the upper face of the above described membrane 11b.

Subsequently, the above described inner container 11 is set on the supporting portion 12a which has been formed at the bottom face of the above described outer container 12, and predetermined amounts of the culture media M are supplied to the insides of the inner containers 11 and the supporting portions 12a of the outer container 12, respectively.

After that, the culture container 4 is transferred to the incubator that is connected to the isolator 2, the incubator is separated from the isolator 2, and the above described cells are cultured for a predetermined period of time. Then, the operations of exchange of the culture medium M and the like are repeated, and thereby the above described cell sheet S can be obtained.

In the meantime, the culture media M circulate between the inside of the above described inner container 11 and each of the supporting portions 12a of the outer container 12 through the membrane 11b of the above described inner container 11, and the culture media M are supplied to the surface and the rear surface of the cell sheet S on the membrane 11b. Thus, it becomes possible that cells are efficiently cultured.

FIG. 3(a) shows an operation of removing the culture medium M from the culture container 4 that is accommodated in the above described isolator 2.

When the cultivation of the cell sheet S in the culture container 4 has been completed, the incubator which accommodates the cell sheet S therein is connected to the isolator 2, and the above described culture container 4 is transferred to the isolator 2. At this time, the lid portion 13 is not mounted on the outer container 12 of the culture container 4, and the above described culture medium M is accommodated in the outer container 12.

From this state, the operator wears the glove 2a of the above described isolator 2, operates suction means such as a pipette, and sucks the culture medium M from the inside of the above described inner container 11 and the supporting portion 12a of the outer container 12. Thereby, the culture medium M is removed which has covered the cell sheet S until then.

FIG. 3(b) shows an operation of mounting the lid portion 13 onto the above described culture container 4, in the isolator 2.

The inside of the above described isolator 2 is kept in the aseptic condition, and accordingly when the lid portion 13 is mounted on the outer container 12 of the above described culture container 4, the internal space which has been sealed by the outer container 12 and the lid portion 13 results in being kept in the aseptic condition.

FIG. 3(c) shows an operation of moving the culture container 4 on which the above described lid portion 13 has been mounted, to the above described cutting device 3.

The above described culture container 4 is taken out to the outside of the isolator 2 through the above described pass box 21 which is not illustrated in FIG. 3(c), and is mounted on the holding table 22 of the above described cutting device 3 in such a state that the above described lid portion 13 is mounted thereon.

At this time, the internal space in the culture container 4 is sealed by the above described lid portion 13, and accordingly the cell sheet S in the culture container 4 is prevented from being contaminated by foreign matters and microorganisms in the external space.

FIG. 4(a) shows an operation of cutting the cell sheet S in the culture container 4 by the above described cutting device 3.

In the above described cutting device 3, the above described laser irradiation means 23 emits the laser L downward; the laser L transmits through the transmission part that constitutes the above described lid portion 13 and then irradiates the cell sheet S in the culture container 4; the above described holding table 22 is moved by the moving means; and thereby the cell sheet S is cut into a desired shape by the laser L.

At this time, the laser L irradiates the cell sheet S from the upper side, and the cell sheet S is in a state of being fixed on the collagen gel layer C; and accordingly the laser L is enabled to cut the cell sheet S with high accuracy.

In addition, the culture medium M in the culture container 4 is already removed, and accordingly the laser L which has transmitted through the lid portion 13 irradiates the cell sheet S without being absorbed by the culture medium M, and can cut the cell sheet S.

The laser L cuts the above described collagen gel layer C also, but transmit through the membrane 11b existing below the collagen gel layer C and the bottom of the outer container 12, and then be absorbed by the above described holding table 22.

Then, after the laser L has cut the cell sheet S accommodated in the required inner container 11, the moving means moves another inner container 11 to a position which is irradiated by the laser L, and then the cutting device repeats the cutting of the cell sheet S.

FIG. 4(b) shows an operation of decontaminating the above described culture container 4 in the pass box 21, in order to transfer the above described cell sheet S which has been already cut to the isolator 2 again.

The above described cutting device 3 is provided in the external space, and accordingly in order that the cell sheet S is cut in the cutting device 3, the above described culture container 4 needs to be taken out to the outside. As a result, foreign matters, microorganisms and the like may attach to the outer surface of the culture container 4.

Then, in order to take out the cell sheet S from the above described culture container 4 in the inside of the isolator 2, it is necessary to decontaminate the outer surface of the culture container 4 so that the internal space of the isolator 2 is not contaminated.

Therefore, the above described culture container 4 is accommodated in the pass box 21, and the outer surface thereof is decontaminated by hydrogen peroxide vapor or the like. Here, the culture container 4 is sealed by the lid portion 13, and accordingly the above described hydrogen peroxide vapor and the like do not enter the inside of the culture container 4, and the cell sheet S is not impaired.

FIG. 4(c) shows an operation of collecting the cut cell sheet S, in the isolator 2.

The culture container 4 which has been decontaminated in the above described pass box 21 is moved into the isolator 2 through the above described connection door 21b, and the above described lid portion 13 is removed in the inside of the isolator 2 which is kept in the aseptic condition.

Subsequently, the operator charges collagenase into the inner container 11 to decompose the collagen gel layer C which has been formed on the upper face of the above described membrane 11b and has held the above described cell sheet S.

Thereby, the cell sheet S which has adhered to the collagen gel layer C until then is isolated, and becomes a state of being simply mounted on the upper face of the above described membrane 11b. Incidentally, the specific operation is described in International Publication No. WO 2012/115244, and accordingly the description will be omitted.

Then, the operator collects the cell sheet S which has been cut into a required shape, by using a picking tool such as a pipette. At this time, the cell sheet S does not adhere to the upper face of the membrane 11b, and accordingly the operator can collect the cell sheet S without damaging the cell sheet S.

According to the above described example, the culture container 4 which accommodates the cell sheet S is sealed by the lid portion 13, and the laser L transmits through the transmission part that constitutes the above described lid portion 13 and then irradiates the cell sheet S; and accordingly it is possible to cut the cell sheet S in a state of having kept the aseptic environment, in the cutting device 3 which is hard to be installed in the aseptic environment.

In addition, a layer which is formed of collagen is provided on the upper face of the membrane 11b of the above described inner container 11, the cell sheet S is cut by the laser L together with the collagen, accordingly the cell sheet S can be cut with high accuracy; in addition, after that, the above described collagen is decomposed by collagenase, and accordingly it is possible to collect the cell sheet S in a state of being isolated from the container.

Incidentally, in the above described example, the culture medium M is discharged from the inner container 11 and the outer container 12, then the lid portion 13 is mounted on the outer container 12, and the outer container 12 is moved to the outside of the isolator 2; but it is also acceptable to take out the inner container 11 which accommodates the above described cell sheet S therein from the outer container 12 which accommodates the culture medium therein, to move the inner container 11 to another outer container 12 which does not accommodate the culture medium M, and to mount the lid portion 13 on the other outer container 12.

In addition, in the above described example, the cell sheet S is cut together with the collagen by the laser L, but it is also acceptable to decompose the collagen layer C in the step concerning FIG. 3(a), and then cut the cell sheet S by the laser L.

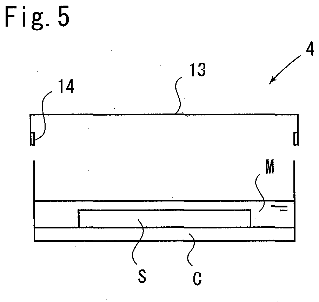

In addition, according to the cutting method of the present invention, if the cell sheet S is cultured in a dish-type culture container 4 as shown in FIG. 5, the culture medium M is removed from the culture container 4, and also the above described lid portion 13 is mounted, the cell sheet S can be cut in the aseptic condition with the use of the cutting method similar to the above described example.

### [Reference Signs List]

1 Cell sheet cutting system
2 Isolator
3 Cutting device
4 Culture container
11 Inner container
11b Membrane
12 Outer container
13 Lid portion
S Cell sheet
L Laser
M Culture medium
C Collagen gel layer

## Claims

1. A method for cutting a cell sheet by moving a container that accommodates the cell sheet therein to a cutting device that emits a laser, and cutting the cell sheet in the container by the laser, **characterized in that** the method comprises:
sealing the container by a lid portion provided with a transmission part made from a material which allows the laser to pass through, in an inside of an aseptic room that has been kept in an aseptic condition;
taking out the container sealed by the lid portion from the aseptic room, and moving the container into the cutting device that is provided in the outside of the aseptic room; and
in the cutting device, passing the laser through the transmission part, and cutting the cell sheet by the laser.

2. The method for cutting the cell sheet according to claim 1, **characterized in that** the method comprises:
accommodating a culture medium in the container, and also culturing the cell sheet by using the culture medium; and
when taking out the container that is sealed by the lid portion from the aseptic room and moving the container to the cutting device, removing the culture medium from the container, in the inside of the aseptic room in advance.

3. The method for cutting the cell sheet according to claim 1 or claim 2, **characterized in that**
the container includes an inner container that can accommodate the culture medium and the cell sheet therein, and an outer container that accommodates the inner container therein, and
when the container is sealed by the lid portion, the lid portion is mounted on the outer container.

4. The method for cutting the cell sheet according to claim 3, **characterized in that**
the inner container includes a membrane in the bottom which passes the culture medium therethrough, and
when the cell sheet is cultured, the cell sheet is cultured on the membrane of the inner container, the culture medium is accommodated in the outer container, and the culture medium is in a state where the culture medium can circulate between the inner container and the outer container through the membrane.

5. The method for cutting the cell sheet according to claim 4, **characterized in that** the method comprises:
providing a layer that is formed of collagen on an upper face of the membrane in the inner container, and culturing the cell sheet on an upper face of the collagen; and
cutting the cell sheet by the laser of the cutting device, moving the container that accommodates the cut cell sheet into the inside of the aseptic room; removing the lid portion from the outer container, then decomposing the collagen of the inner container by collagenase, and taking out the cut cell sheet, from the inner container.
